# EUROPEAN PATENT APPLICATION

(11) **EP 1 908 403 A1**
(43) Date of publication of application: **09.04.2008**
(21) Application number: 06767798.9
(22) Date of filing: 04.07.2006
(51) Int. Cl.: A61B 5/055, A61B 6/04

(54) **POSTURE CHANGING DEVICE OF MEDICAL IMAGE DIAGNOSING DEVICE**

(30) Priority: 11.07.2005 JP 2005201423
(71) Applicant: Hitachi Medical Corporation, Chiyoda-ku Tokyo (JP)
(72) Inventor: MATSUSHITA, Taisuke c/o HITACHI MEDICAL CORP., Tokyo 1010047 (JP); SATO, Yutaka c/o HITACHI MEDICAL CORP., Tokyo 1010047 (JP); HIRAMATSU, Kazuaki c/o HITACHI MEDICAL CORP., Tokyo 1010047 (JP); KONAMI, Makoto c/o HITACHI MEDICAL CORP., Tokyo 1010047 (JP); ASAI, Shoujirou c/o HITACHI MEDICAL CORP., Tokyo 1010047 (JP)
(74) Representative: Strehl, Peter
(86) International application number: PCT/JP2006/313267
(87) International publication number: WO 2007/007583

(57) **Abstract**

A postural change device used to allow imaging of a plurality of postures while changing the posture of a portion to be examined of an object, by means of a medical image diagnostic apparatus.

The postural change device comprises a head-use balloon and a trunk-use balloon dispersedly arranged in a bag-form mat, and a compressor and a valve body carrying out parallel actions on the two balloons, that is, maximum expansion medium expansion complete contraction on the head-use balloon, and complete contraction medium expansion maximum expansion on the trunk-use balloon, whereby it is possible to sequentially change the cervical spine of the object from an anteversion posture to a neutral posture to a retroflexed posture.

## Description

### Technical Field

The present invention relates to a postural change device for the usage of imaging while changing the body-posture of an object by a medical image diagnostic apparatus such as an X-ray CT apparatus or MRI apparatus.

### Background Art

One of the postural change devices in a conventional medical image diagnostic apparatus is, for example, a head-reception part provided on the top-plate of a table on which an obj ect is placed for a device such as an X-ray CT device. This type of head-reception part has a configuration wherein a head-reception part for placing a head is placed on the head-reception holder, and a circular-arc shaped concave is formed on the upper part of the head-reception holder. Also, the bottom surface of the head-reception part is provided with a circular-arc shaped convex. By such configuration, it is possible to continuously adjust tilt-angle with respect to the head-reception holder (For example, refer to Patent Document 1).

Also, there is a type of X-ray imaging apparatus comprising a top-plate rolling mechanism for rotating the body of the object centering on the body axis. Even if an object is not capable of maintaining a predetermined tilt-angle centering on the body axis on the flat surface of a top-plate, comprising a rolling top-plate enables rotation of an object to a predetermined angle centering on the body axis and maintaining the rotated angle, through operation of the rolling mechanism of the top plate by an operator (refer to Patent Document 2).

Meantime, Patent Document 3 and Patent Document 4 disclose a technique related to an air mat for preventing bedsores of chronically bedridden patients who are not capable of moving their bodies by themselves such as bedridden elderly people or severely ill patients, which is not for use in combination with medical image diagnostic apparatuses.
Patent Document 1: JP-A-2002-291731
Patent Document 2: JP-A-H10-201756
Patent Document 3: JP-A-H8-52180
Patent Document 4: JP-A-H8-206159

### Disclosure of the Invention

### Problems to be Solved

In the postural change device disclosed in the above-mentioned Patent Document 1, it is necessary to adjust the tilt-angle of the head-reception part manually, which leaves a problem to be solved such as in the case of imaging a neck region of the object at a plurality of flexed angles that the adjustment of the tilt-angle of the head-reception part has to be made by the operator at each step of the imaging according to the weight of the head of the object, whereby lowering the operation efficiency upon imaging.

Also, when an attempt is made to use a head-reception part made for the X-ray CT device upon imaging cervical spine of the object with an MRI apparatus which is preferable for imaging the cervical spine, the head-reception part can not be tilted toward the lower side of the top-plate, that is bending the cervical spine of the object backward, since the space between the top-plate of the table and a magnet or a gantry of the MRI apparatus is too small.

Further, when an attempt is made to apply the top-plate rolling mechanism of the X-ray apparatus disclosed in Patent Document 2 to an X-ray CT apparatus or MRI apparatus, since the diameter of the gantry opening is limited, it leaves the problem that the postural change of the object can not be performed appropriately.

Also, the air mat disclosed in Patent Document 3 and Patent Document 4 is difficult to use in the small imaging space of MRI apparatus since it has two-layer structure consisting of the main air mat by which the object is contacted and the postural change air mat to be placed under the main air mat. It also presents a difficulty when used as a part of an X-ray imaging apparatus, since the penumbrae generated in the images will be large whereby interfering from the acquisition of preferable images. When attempting to use the two-layer structure air mat for the imaging diagnostic apparatus, it causes difficulty in performing a desired postural change, since depending on the region for examination the information on postural change given to the postural change air mat can not be directly transmitted to the object due to the existence of the main air mat between the postural change air mat and the object.

The object of the present invention is to solve the above-described problems by providing a postural change device for a medical image diagnostic apparatus capable of changing the posture of the object with ease upon imaging by a medical image diagnostic apparatus and improving the operation efficiency thereof.

### Means to Solve the Problems

The postural change device of the present invention for the medical image diagnostic apparatus comprises:
an expansion/contraction body placed on the table of the medical image diagnostic apparatus, for changing the posture of an object to be examined by expanding/contracting through the supply/discharge of a fluid while the object is placed on the table;
a fluid supply source for supplying fluid to the expansion/contraction body; and
a control unit for adjusting or controlling the supply/discharge of the fluid for the expansion/contraction body.
The preferable configuration of postural change device of the present invention is such that a plurality of expansion/contraction bodies are dispersedly arranged on the table, and controlling of the respective expansion/contraction bodies is performed independently or at the same time by the control unit.

### Brief Description of the Diagrams

Fig. 1 is a block diagram showing the general configuration of a first embodiment of a postural change device for a medical image diagnostic apparatus of the present invention.
Fig. 2 shows internal structure of a postural change mat illustrated in Fig. 1.
Fig. 3 shows the head-use balloons incorporated in a postural change mat viewed from the direction of an arrow "A" in Fig. 2, showing the condition of expansion and contraction.
Fig. 4 is a plain view of the trunk-use balloons incorporated in the postural change mat, and expansion/contraction condition thereof viewed from the direction of an arrow "B" of the plain view.
Fig. 5 is a plain view showing another embodiment of a trunk-use balloon.
Fig. 6 is a plain view of an operation panel incorporated in the postural change mat shown in Fig. 2.
Fig. 7 is a pneumatic circuit diagram of the first embodiment.
Fig. 8 shows correspondence between the operating order of the postural change device in Fig. 1 and postural change of the cervical spine of an object.
Fig. 9 is a block diagram of a general configuration of a second embodiment of the postural change device related to the present invention.
Fig. 10 is a block diagram showing a configuration of a mat controller of the postural change device shown in Fig. 9.
Fig. 11 is a time chart showing the order of imaging motion of the cervical spine of the object by the postural change device of the second embodiment and an MRI apparatus.
Fig. 12 shows another embodiment of the postural change mat.
Fig. 13 shows operation of the postural change mat illustrated in Fig. 12 and the postural change condition of the object.
Fig. 14 shows yet another embodiment of the postural change mat.
Fig. 15 shows yet another embodiment of the postural change mat.
Fig. 16 shows yet another embodiment of the postural change mat.
Fig. 17 shows yet another embodiment of the postural change mat.
Fig. 18 shows yet another embodiment of the postural change mat.
Fig. 19 shows yet another embodiment of the postural change mat.

### Best Mode for Carrying Out the Embodiment

Hereinafter, the best mode for carrying out the present invention will be described referring to the diagrams.

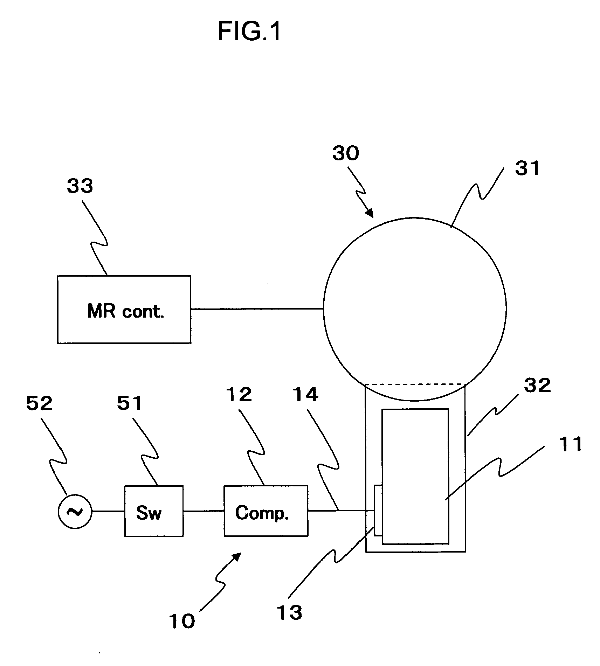

Fig. 1 is a block diagram showing a general configuration of a first embodiment of the postural change device for a medical image diagnostic apparatus related to the present invention. The present embodiment illustrates an example of a postural change device of a manual operation type for imaging the examination region while changing posture of an object using an MRI apparatus as a medical image diagnostic apparatus. The MRI apparatus 30 comprises:
a gantry 31 comprising a magnet as a magnetic field generator, a gradient magnetic field coil and a transmission coil;
a table 32 for placing the object on which a reception coil is applied, and for moving the object to an imaging position; and
an MR controller 33 for operating the MRI apparatus. Out of these components of the MRI apparatus, the gantry 31 and the table 32 are placed in an examination room, and the MR controller 33 is placed in an operators room. Though a gradient magnetic field source unit and a storage unit for storing measurement data and images are also provided to the MRI apparatus 30, diagrammatic representation is omitted since they have little relation to the description of the present invention.

Postural change device 10 of the present embodiment consists of:
a postural change mat (main body of the postural change device) 11;
a compressor 12 for carrying out supply/exhaust of air to/from the postural change mat 11;
a valve unit 13 provided at closely to the postural change mat 11; and
an aeration/emission tube 14 for connecting between an intake/exhaust vent of the compressor 12 and the valve unit 13. Postural change mat 11 is placed on a top-plate of the table 32 of the MRI apparatus 30. Compressor 12 is connected to a power source 52 via an on/off switch 51 of the power source.

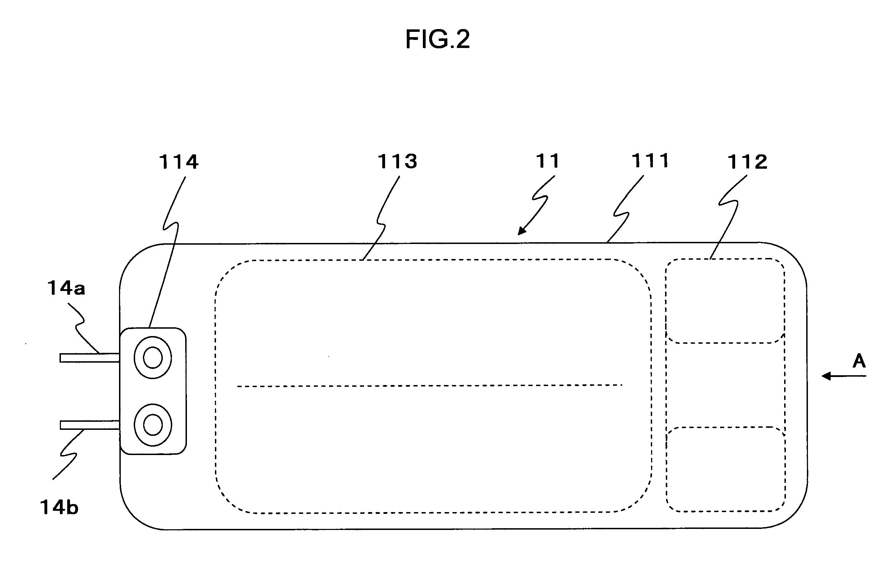

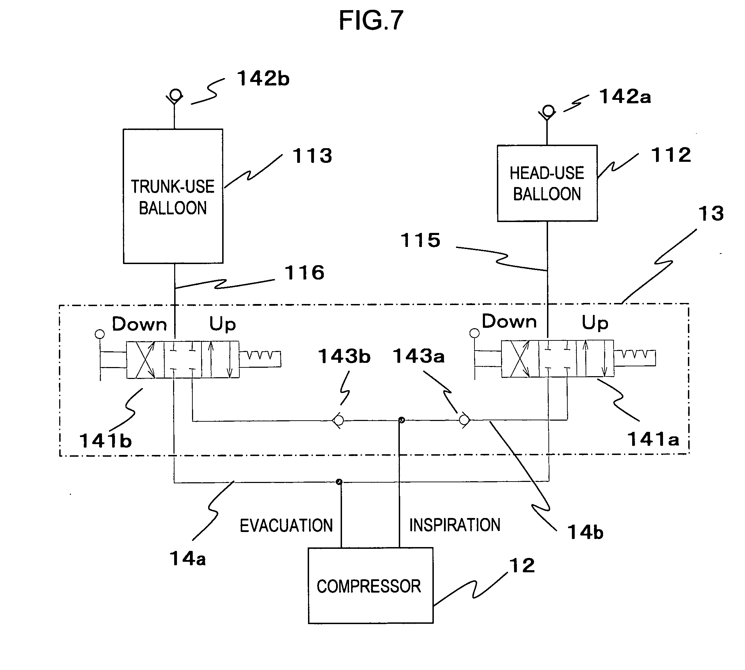

Next, the configuration of the postural change mat 11 will be described in detail. Fig. 2 shows an internal configuration of the postural change mat 11. Postural change mat 11 consists of:
a bag-form mat 111;
a head-use balloon 112 firmly fixed on one side of internal surface of the mat 111 using material such as adhesive tape or adhesive bond;
a trunk-use balloon firmly fixed on a central part of the internal surface of mat 111 using material such as adhesive tape or adhesive bond, having a predetermined distance from the head-use balloon 112;
an operation panel 114 disposed at the end portion of the longitudinal direction of the mat 111;
a valve unit 13 disposed on the back surface of the operation panel 114;
an aeration/emission tube 115 for connecting between the head-use balloon 112 and the valve unit 13 (refer to Fig.7); and
an aeration/emission tube 116 for connecting between the trunk-use balloon 113 and the operation panel 114 (refer to Fig.7). A pair of aeration/emission tubes 14a and 14b (refer to Fig.7) are drawn from the valve unit 13 to the outside.

The mat 111 is formed into a cuboid-formed mat having a predetermined dimension using a fabric being flexible and easy to dry, or resin film such as polyvinyl chloride resin, polyethylene resin and polyurethane resin. The head-use balloon 112 and the trunk-use balloon 113 disposed inside of the mat 111 are formed into balloon-like form also using resin film being flexible such as polyvinyl chloride resin, polyethylene resin and polyurethane resin in the same way as the mat 111.

Next, detailed explanation on the above-mentioned balloons will be described.

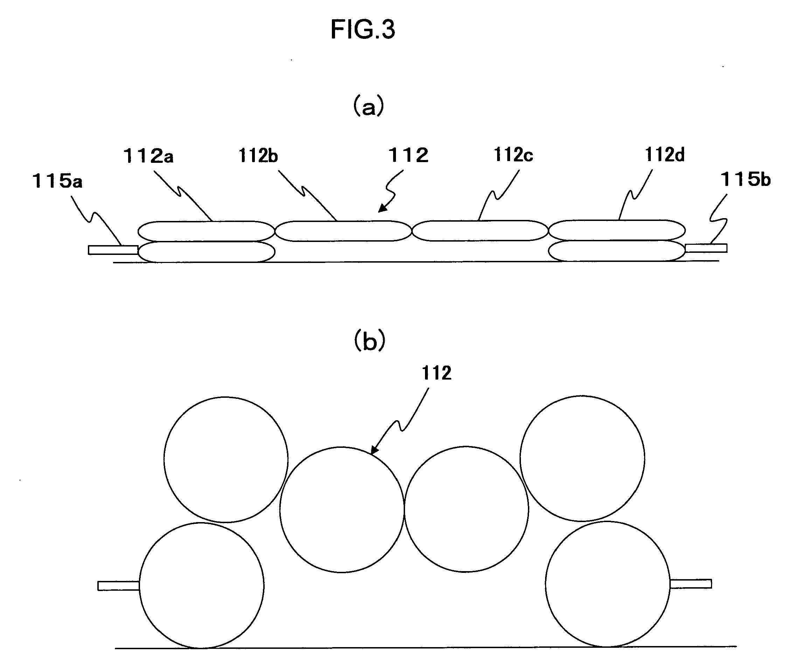

Fig. 3 shows a head-use balloon 112 in exhaust (contracted) condition viewed from the direction of an arrow "A" in Fig. 2. The dimension is set for the head-use balloon 112 in the body-axis direction so that the head of the object can be supported. As shown in Fig. 3 (a), the head-use balloon 112 is formed by four balloon blocks 112a, 112b, 112c and 112d in the minor-axis direction of the mat, and is formed symmetrical to the center of the minor axis direction of the mat. The balloon blocks 112a and 112d positioned on the long side of the mat are formed by a two-layer balloons, and the balloon blocks 112b and 112c positioned on the inner side thereof are formed by a single-layer balloons. It is then configured so that the balloons positioned on the upper side of the two-layered balloon blocks 112a and 112d are connected to the balloon blocks 112b and 112c. The head-use balloon 112 configured as above is formed as one body so the air can get through inside, and the aeration/emission tubes 115a and 115b are connected to the lower part of the respective balloon blocks 112a and 112d. Fig. 3 (b) shows a condition in which the head-use balloon 112 is expanded approximately to the maximum. As it is understandable by referring to Fig. 3 (a) and Fig. 3 (b), the balloon blocks 112b and 112c are elevated when the balloon blocks 112a and 112d are expanded. Though the elevated the balloon blocks 112b and 112c are suspended in midair, they are at the position that is lower than the top position of the balloon blocks 112a and 112d on both sides. Therefore, the head of the object is stably supported since the displacement of the head in lateral direction is prevented by the balloon blocks 112a and 112d when the head-balloon 112 is expanded.

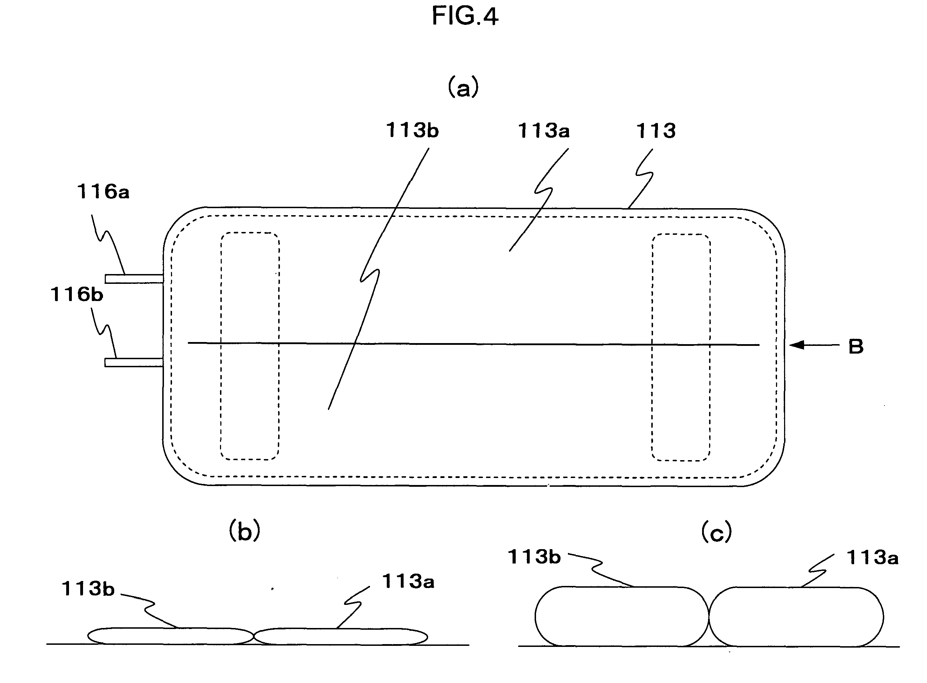

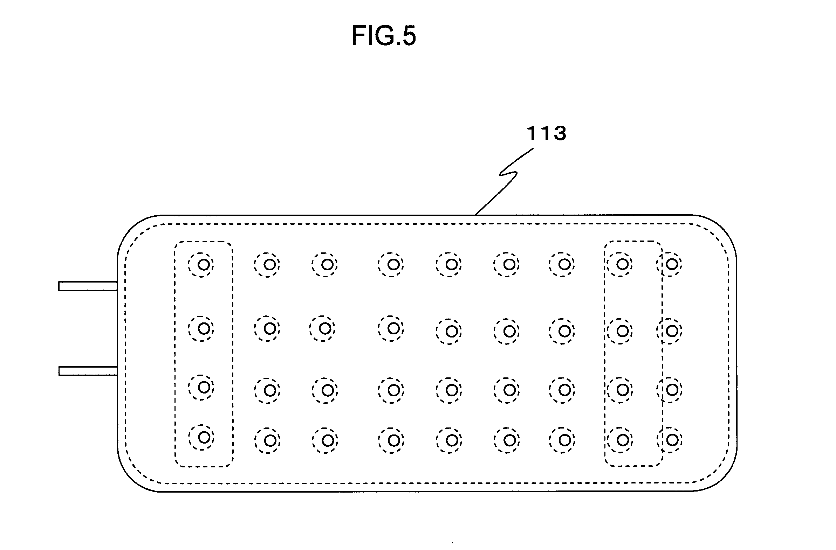

Fig. 4 is a diagram wherein the trunk-use 113 is extracted from Fig. 2. Fig. 4 (a) is a plain view thereof, and Fig. 4 (b) and Fig. 4 (c) are the diagrams viewed from the direction of an arrow "B" in Fig. 4 (a). The dimension is set for the trunk-use balloon 113 in the body-axis direction and in the direction orthogonal to the body-axis direction, so that from the shoulder to the legs of the object can be placed. As shown in Fig. 4, the trunk-use balloon 113 is divided into at least two chambers 113a and 113b in minor-axis direction. The reason for dividing the trunk-use balloon 113 into two in minor-axis direction is that, when air is supplied in the balloon (refer to Fig. 4 (c)), to support the object stably by forming a concave in the center part through dividing the balloon into two chambers, since the object cannot be stably supported if the balloon expands in singular arc-form at the cross-section in minor-axis direction. Therefore, the number of divisions for the trunk-use balloon in minor-axis direction does not have to be limited to 2, but 3 or more may be considered. Also, the division of the balloon does not have to be performed in fully expanded condition. To the chambers 113a and 113b of the trunk-use balloon 113 configured as above, the aeration/emission tubes 116a and 116b are connected. As for the form of the trunk-use balloon 113 for stably supporting the object upon expanding the balloon, other than dividing the balloon into a plurality of chambers as described above, it may be formed so that concavities and convexities are formed as close as a bed for sleeping upon expanding the balloon by distributing a plurality of connecting points of the upper and lower membranes over the entire surface of the upper and lower membranes of the balloon as shown in Fig. 5.

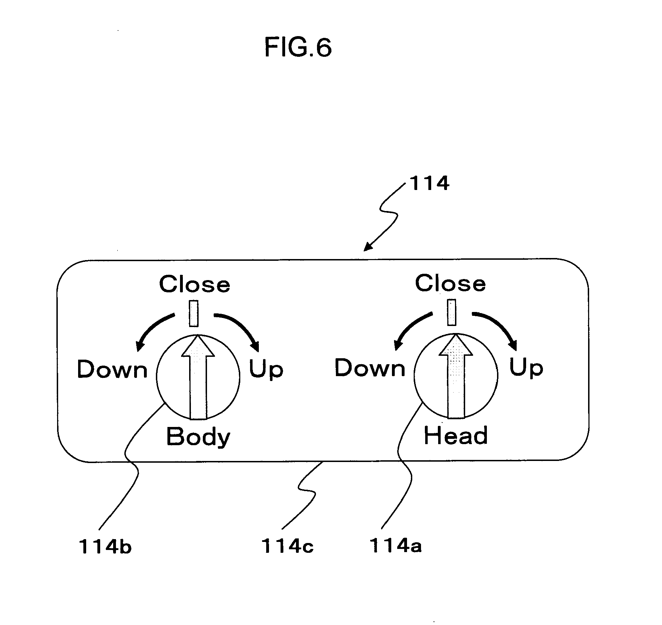

Next, the operation panel 114 and the valve unit 13 provided to the mat 111 will be described. As shown in Fig. 2, the operation panel 114 is disposed at the end of the mat at which the feet of the obj ect laid on the mat 111 are positioned. The operation panel 114 has an operating device of the valve unit, that is a valve control button 114a for controlling supply/exhaust of air to/from the head-use balloon 112, a valve control button 114b for controlling supply/exhaust of air to/from the trunk-use balloon 113, and a panel 114c for indicating the operation of the valves by marking. These valve control buttons 114a and 114b are provided at the end of a plunger of the valves, as shown in Fig. 6.

Fig. 7 is an air circuit diagram for controlling supply/exhaust of air for the two mats by operating the buttons on the above-mentioned operation panel 114. As shown in Fig. 7, the air circuit of the present embodiment is configured by connecting the compressor 12, 3 stage valves 141a and 141b, safety valves 142a and 142b, the head-use balloon 112 and the trunk-use balloon 113 by tubes. More specifically, two 3-stage valves 141a and 141b are connected in parallel with the intake vent and the exhaust vent of the compressor 12, and the head-use balloon 112 and the trunk-use balloon are connected to either the intake valve or the exhaust valve of each 3 stage valves 141a and 141b. Then the check valves 143a and 143b are provided to a piping tube between the intake vent of the compressor 12 and the two 3-stage valves 141a and 141b as shown in the diagram. The above-mentioned 3-stage valves perform the connection to the air supply vent and the exhaust vent of the compressor, full-closure of the vents, and shift/connection of the intake vent and the exhaust vent of the compressor, by switching the rotational positions to the 3 choices of "Right", "Mid" and "Left" of the plunger respectively. Also, check valves 143a and 143b are the valves for making air from the head-use balloon 112 and the trunk-use balloon 113 to be distributed only to the intake valve direction of the compressor 12. The safety valves 142a and 142b are also provided to the head-use balloon 112 and the trunk-use balloon 113. These safety valves 142a and 142b are to be opened when the pressure inside of the balloons reaches more than a predetermined value for preventing the pressure from surpassing the predetermined value, in order to protect the balloons from being damaged. As for the parts to be provided for the operation panel unit including the valves, non-magnetic material is used to avoid disturbance of magnetic field to be generated from the magnetic field generating device of the MRI apparatus 30. The valves 141a and 141b shown Fig. 7 are both in full-closure condition.

The above-mentioned valves 141a and 141b are attached on the backside of the plate surface of the operation panel 114. On the right surface of the plate surface 114c of the operation panel 114, letters and arrows for indicating the operating direction of the balloons operated by the valves 141a and 141b, that is the operation related to elevation (Up), descension (Down) and shutoff (closure of the valves: Close) are printed or engraved.

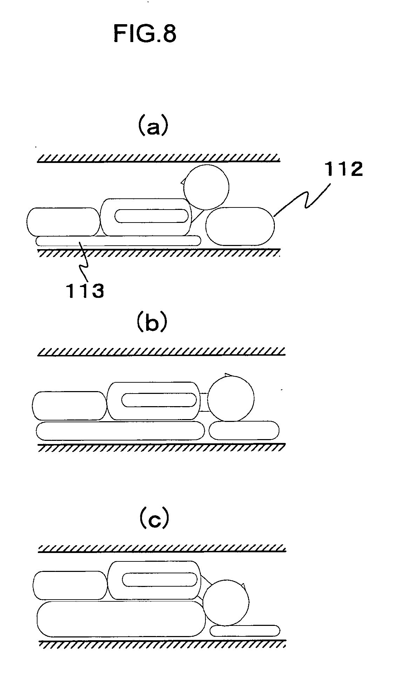

Next, operation method of the above-configured postural change device 10 will be described along with imaging procedure for the cervical spine of an object by the MRI apparatus 30.
First, the operator lays an object having injured cervical spine on the postural change mat 11 placed on the table 32 positioned where the object gets on/off. At this time, the object is laid so that the head of the object is positioned on the head-use balloon 112. In the case that the table 32 has a mat on it to be used for usual MRI imaging, the mat is to be removed as appropriate. Next, the table is moved through the operator so that the cervical spine of the object will be positioned approximately at the center of the measurement space of the MRI gantry. When the movement of the obj ect is completed, MR imaging is performed while sequentially bending the cervical spine of the object forward/backward as shown in Fig. 8 (a) ~ Fig. 8 (c).

First, as shown in Fig. 8 (a), the imaging is performed at a position that the cervical spine of the object is bent forward. When the operator injects a power source 52 by turning a switch 51 on, the compressor 12 starts operating. Then the head-use balloon 112 is inflated through the valve operation via the operation panel 114 provided to the postural change mat 111 by the operator. The valve operation for inflating the head-use balloon 112 is performed by rotating the valve control button 114a which is attached to a plunger of the valve 141a wherein the air supply/exhaust to/from the balloon 112 is in the closed condition, to the right ("Up") direction. Upon supplying air to the head-use balloon 112 , the operator performs valve operation by switching the valve control button 114a between "Up" and "close" while observing the head position of the object gradually bending forward. Then the operator turns the valve control button 114a to "Close" when it is determined that the cervical spine of the object has reached to the imaging angle of an anteversion posture. By doing so, air supply to the head-use balloon 112 is interrupted and the pressure increase in the balloon is suspended, whereby maintaining the cervical spine of the object at the imaging angle of the anteversion posture. The operator then performs the imaging of the cervical spine by the MRI apparatus 30 through operating the MR controller 33 in the condition that the cervical spine of the object is maintained at the anterversion angle.

When the imaging is completed, the preparation is performed by the operator for MR imaging at the second imaging angle wherein the cervical spine of the object is lowered and is, for example, parallel to the body axis direction (intermediate position) as shown in Fig. 8 (b) by again operating the operation panel of the postural change device 10. The operation for lowering the angle of the cervical spine of the obj ect is performed by lowering the head of the obj ect being in the anteflexed angle and raising the trunk of the body. This operation is implemented by rotating the valve control button 114a of the valve 141a toward "Down", and the valve control button 114b of the valve 141b toward "Up" . More specifically, when the valve control button 141a is turned to "Down", the aeration/emission tube of the head-use balloon 112 is connected to the intake vent of the compressor 12, whereby opening the check valve 143a and sucking the air out of the balloon by the aspiration of the compressor 12. On the other hand, when the valve control button 141b is turned to "Up", the aeration/emission tube of the trunk-use balloon 113 is connected to the exhaust vent of the compressor 12, whereby supplying air to the balloon from the compressor 12. The operator adjusts the cervical spine of the object to the position (angle) parallel to the body-axis as shown in Fig. 8 (b), by properly operating the two valve control buttons. Then the valve control buttons 114a and 114b are turned to "Close" when the operator determines that the cervical spine of the obj ect has reached the angle parallel to the body axis. By doing so, supply/exhaust of air to the head-use balloon 112 and trunk-use balloon 113 is interrupted and increase/decrease of pressure in the balloon is suspended, whereby maintaining the angle of the cervical spine of the object parallel to the body axis. The operator then performs the imaging of the cervical spine by the MRI apparatus 30 through operating the MR controller 33 in the condition that the cervical spine of the object is maintained parallel to the body axis.

When the imaging is completed, the preparation is performed by the operator for MR imaging in the third imaging angle wherein the cervical spine of the object is bent backward as shown in Fig. 8 (c) by again operating the operation panel of the postural change device 10. The operation for bending the cervical spine of the object backward is performed by deflating the head-use balloon 112 from the condition that the head-use balloon 112 and the trunk-use balloon 113 are both expanded to the intermediate thickness, and further expanding the trunk-use balloon 113. More specifically, when the valve control button 141a being in the closed condition is turned to "Down" and the valve control button 141b being in the closed condition is turned to "Up", air in the head-use balloon 112 is further sucked out by the compressor 12 and air is further supplied to the trunk-use balloon 113. At this time also the operator gradually changes the angle of the cervical spine of the object through the valve operation while carefully observing the patient. Then the valve control buttons 114a and 114b are turned to "Close" when the operator determines that the cervical spine of the object reached the angle for imaging in a retroflexed position. By doing so, supply/exhaust of air to the head-use balloon 112 and trunk-use balloon 113 is interrupted and increase/decrease of pressure in the balloon is suspended, whereby maintaining the cervical spine of the object at a retroflexed angle. The operator then performs the imaging of the cervical spine by the MRI apparatus 30 through operating the MR controller 33 in the condition that the cervical spine of the object is maintained at a retroflexed angle.

While the first embodiment of the present invention is described above using the example for imaging diagnostic images by changing the angle of cervical spine of the obj ect in 3 steps, it is needless to say that the angle of the cervical spine can be set in arbitrary number of angles by properly controlling valves 114a and 114b. Also, since the postural change device of the present embodiment is manually operated by the operator while carefully observing the object laid at the imaging position of the image diagnostic apparatus at his/her side, the operator is able to give the patient a sense of security and be sensitive to the signals of discomfort or pain coming from the patient.

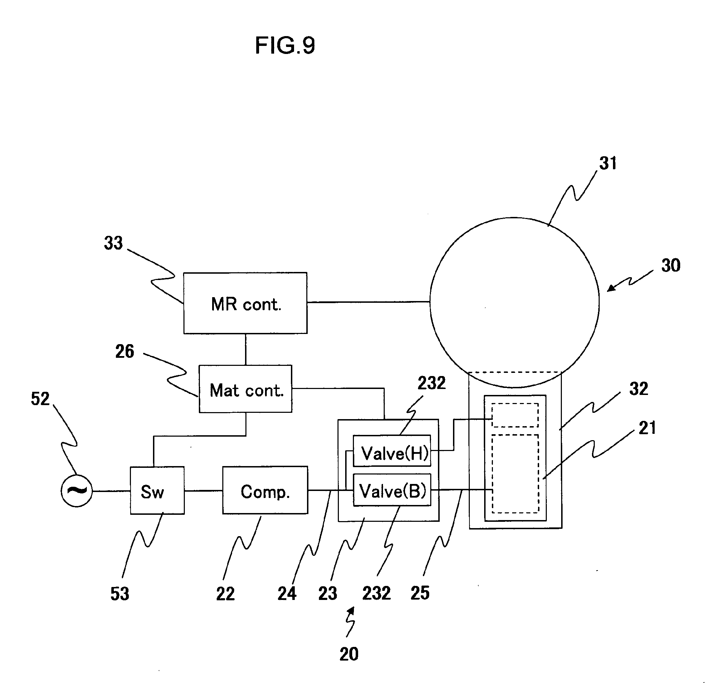

Next, the second embodiment of the present invention will be described referring to Fig. 9. In the second embodiment of the present invention, the postural change device described in the first embodiment is changed from a manual operation type to an automatic operation type capable of automatically and simultaneously operating with the image diagnostic apparatus. Therefore, the postural change mat itself consists of head-use balloon 112 and the trunk-use balloon 113 as is the first embodiment, but the 3-stage valves that are manual valves in the first embodiment are changed to electromagnetic valves. In Fig. 9, 31 indicates a gantry including a static magnetic field generating device of the MRI apparatus 30, 32 indicates a table for placing an object and 33 indicates an MR controller. In addition, the MRI apparatus 30 includes, other than the above-described components, devices such as a gradient magnetic field source, a transmission/reception coil, a monitor and a storage device that are omitted in the diagram. With such configured MRI apparatus 30, a postural change device 20 configured as below is interlocked. The postural change device 20 consists of a postural change mat 21, a compressor 22, a valve unit 23 comprising a 3-stage electromagnetic valve 231 for controlling air supply/exhaust to/from the head-use balloon 112 and a 3-stage electromagnetic valve 232 for controlling air supply/exhaust to/from the trunk-use balloon 113, a postural change mat controller 26 (hereinafter referred to as a mat controller) placed in the operators room of the MRI apparatus, aerator/emission tubes 24 and 25, power source 52 and an on/off switch 53 of the compressor.

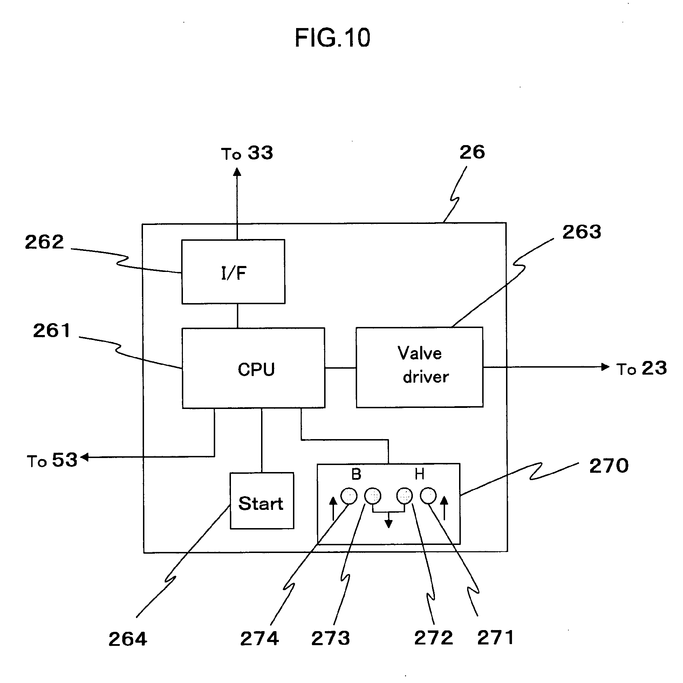

The mat controller 26 comprises, as shown in Fig. 10, CPU 261 for controlling a control unit of the postural change mat 21, an interface (I/F) 262 for exchanging the signals to/from the MR controller 33, a valve drive circuit 263 for outputting the signals for driving the valve unit 23, an imaging preparation starting switch 264 for inputting the command to start preparation for joint motion imaging by driving and operating the MRI apparatus 30 and the postural change device 20 at the same time. Also, a manual operation panel 270 of the postural change mat 21 is provided to the mat controller 26. Software for interlocking the operation of the pulse sequence for joint motion imaging in the MRI apparatus 30 and the operation of the postural change device 20 is also installed in CPU 261.

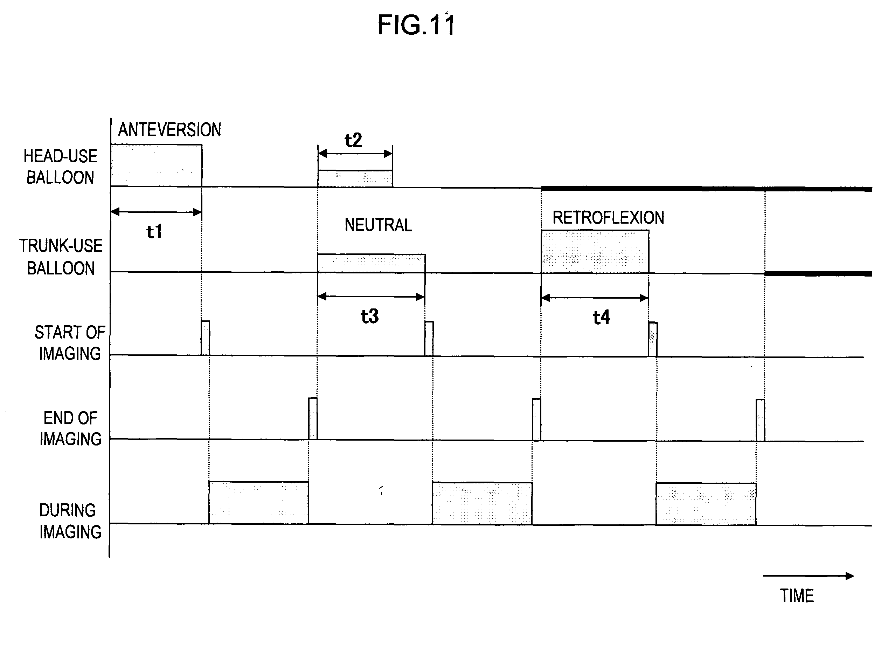

Next, the operation will be described for the case of performing an MR imaging of kineticism of cervical spine of an object in the same manner as embodiment 1 using the postural change device 20 of the present embodiment. Fig. 11 is a time chart for imaging kineticism of cervical spine of the object by automatically operating the postural change device 20 of the present embodiment and the MRI apparatus 30 at the same time.

Prior to the imaging, the object is placed on the table 32 on which the postural change mat is placed, and the cervical spine of the object is moved to approximately the center of measurement space in the gantry 31. The operator attaches a reception coil for measuring MR signals to the cervical spine of the patient, and inputs the command for preparation to start imaging from imaging preparation starting switch 264 of the mat controller 26 after the completion of the imaging preparation. When the command for preparation to start imaging is inputted to the mat controller 26, the CPU 261 that received the command outputs the operation command to the switch 53 and the valve drive circuit 263 for operating the mat 21. By this command, switch 53 is turned on, compressor 22 is operated, and the electromagnetic valve 231 is operated so that air is supplied to the head-use balloon 112.

A time (t) for adjusting the positioning of the cervical spine of the object to a predetermined position by supplying air to the balloon or discharging air from the expanded balloon can be calculated by measuring it in advance or from the aeration/emission amount per unit time of the compressor 22. Therefore, elapsed time from starting air supply to the head-use balloon 112 is measured by a timer provided to CPU 261, and the command to close the valve 232 is outputted from the CPU 261 to the valve drive circuit 263 at the point the set time (tl) has been elapsed. The CPU 261 may stop operation of the compressor at the point the time t1 has been elapsed. Also at the same time, a signal for completion of imaging preparation is outputted from the CPU 261 to the MRI controller 33 via the I/F 262. This signal for completion of imaging preparation means that the head-use balloon 112 is expanded and the cervical spine of the object has reached an anteflexed state. The MRI controller 33 which received the signal for completing the imaging preparation drives pulse sequence for the imaging. In the case, as an example, that the pulse sequence is a gradient echo (GE) method, the imaging is performed by the procedure described below. That is, gradient magnetic field is generated in the imaging slice direction from the gradient magnetic field coil provided in the gantry 31, and the RF pulses having the predetermined frequency band for imaging the cervical spine of the object are irradiated to the object from the irradiation coil. By doing so, nuclear spin in the slice (cross-section) of the predetermined thickness of the cervical spine of the object is excited. Next, the gradient magnetic field of the predetermined quantity is applied in phase encode direction as well as the gradient magnetic field in readout direction is applied, and the excited nuclear spin is dispersed. After that, under the application of the readout gradient magnetic field of which the polarities are reversed, NMR signals (echo signals) are measured via the reception coil. The application quantity of the phase encode gradient magnetic field is varied stepwise by this operation, and the stepwise variation is repeated for the predetermined number of times, for example, 256 times. By such procedure, imaging of the cervical spine in anteversion condition is completed.

When the imaging is completed, an imaging completion signal is outputted to the mat controller 26 from the MRI controller 33. Then the CPU 261 outputs the control signals for supplying/ exhausting air to/from the postural change mat 21 to the valves 231 and 232, in preparation for the next imaging of the cervical spine in the intermediate position. The valve 231 driven by the control signal from the CPU 261 connects the head-use balloon 112 to the intake vent of the compressor 22, and the valve 232 connects the trunk-use balloon 113 to the exhaust vent of the compressor 22. After the input of the control signal, the valves 231 and 232 are controlled by the CPU 261 so that the air can be discharged from the head-use balloon 112 and supplied to the trunk-use balloon 113 only for the time t2 and the time t3 (t3 < t2) respectively. In other words, the valves 231 and 232 receive the total-closure signal from the CPU 261 after the times t2 and t3. The CPU 261 also may stop the operation of the compressor 22 after the time t3. As a result, the cervical spine is maintained in the intermediate posture. Then the CPU 261 transmits the completion signal to the MRI controller 26 for the imaging preparation of the cervical spine in the intermediate posture after the time t3.

In the same way as the imaging in the anteversion posture, the MRI controller 33 which has received the signal from the mat controller 26 for completing the imaging preparation for the cervical spine in the intermediate posture, applies a slice selecting gradient magnetic field, an RF pulse for excitation, a phase encode gradient magnetic field and a readout gradient magnetic field in conformity to a GE method pulse sequence, and repeats to execute the GE method pulse sequence while varying the phase encode gradient magnetic field stepwise 256 times in total in the same way as previously described. When the GE method pulse sequence is executed 256 times, the imaging of the cervical spine in the intermediate posture is completed.

When the imaging is completed, an imaging completion signal is outputted from the MRI controller 33 to the mat controller 26. Then the CPU 261 outputs the control signal for supplying/exhausting air to/from the postural change mat 21 to the valves 231 and 232 in preparation for the next imaging of the cervical spine in the retroflexion posture. By the control signal from the CPU 261, the valve 231 connects the head-use balloon 112 to the intake vent of the compressor 22, and the valve 232 connects the trunk-use balloon to the exhaust vent of the compressor 22. As a result, air in the head-use balloon 112 is discharged and it reaches the completely deflated state after time t4 from the exhaust starting time. On the other hand, the trunk-use balloon 113 reaches the completely expanded state after time t5 (t5 > t4) from the exhaust starting time. After the time t4 and the time t5, the valves 231 and 232 are switched to totally closed state by the signal from the CPU 261. Accordingly, the cervical spine of the object is maintained in the retroflexion posture. Then the CPU 261 transmits the signal of completion for imaging preparation of the cervical spine in the retroflextion position, to the MRI controller 33 after the time t5. At this time also, the CPU 261 may stop the operation of the compressor 22.

The MRI controller 33 which has received the signal from the mat controller 26 for completing the imaging preparation for the cervical spine, in the same manner as the imaging in the anteversion and intermediate position, applies a slice selecting gradient magnetic field, an RF pulse for excitation, a phase encode gradient magnetic field and a readout gradient magnetic field in conformity to a GE method pulse sequence, and repeats to execute the GE method pulse sequence while varying the phase encode gradient magnetic field stepwise 256 times in total in the same way as previously described. When the GE method pulse sequence is executed 256 times, the imaging of the cervical spine in the retroflexion posture is completed. Upon completion of the above-described imaging in anteversion, intermediate and retroflexion postures, a series of imaging of the cervical spine is concluded. When the imaging is ended, a completion signal of the imaging is outputted from the MRI controller 33 to the mat controller 26. The CPU 261 that has received the imaging completion signal outputs the signal to the valve unit 23 for exhausting all the air in the postural change mat 21. By this signal, the air in the trunk-use balloon 113 is completely exhausted, and the object on the table 32 is laid on the table in supine position. Then upon the table 32 is moved to the outside of the gantry 31 by the operator, imaging test on kinematic motion of the cervical spine of the object is completed. After the completion of imaging, interpretation of the image will be performed by a doctor.

Though the above-described second embodiment is configured so that the kinematic motion image test of the object by an image diagnostic apparatus and postural change device is to be started by the command through the starting switch for imaging preparation provided to the postural change device, the present invention does not need to be limited to such embodiment, and the software may be changed to be initiated by the image diagnostic apparatus.

In the mat controller 26 of the present embodiment, a manual operation panel 270 for manually operating the postural change mat 21 as shown in Fig. 10. To the manual operation panel 270, expansion switch 271 and contraction switch 272 are provided for the head-use balloon 112, and expansion switch 273 and contraction switch 274 are provided for the trunk-use balloon 113. These switches are for the operator to use in the case that clinical condition of the object is serious and the posture of the object needs to be changed intermittently, and the postural change mat can be operated basically in the same manner as the embodiment 1. Also, in the case of operating these switches, manual operation of the valve should be set to prevail over the automatic operation. As means for that, software for prioritizing the manual operation of the valves can be loaded to CPU 261.

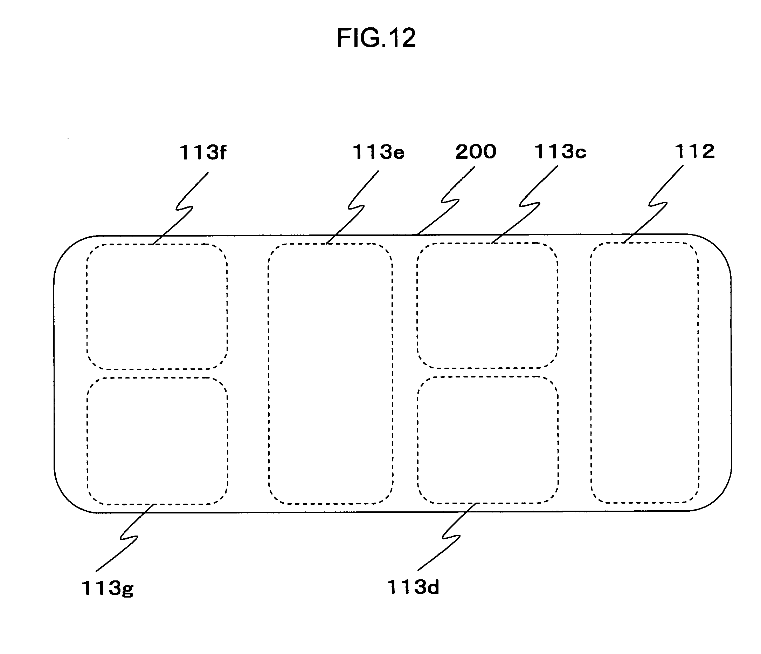

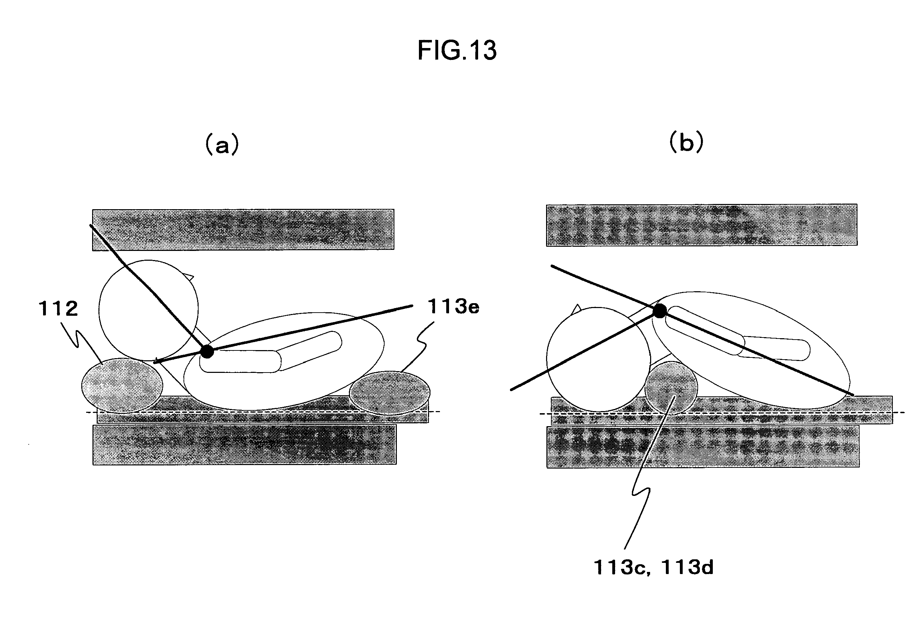

While the example has been described in the above-mentioned embodiments that the postural change mat is configured such that one each of the head-use balloon and the trunk-use balloon is disposed inside of the mat, the present invention is not to be taken by way of limitation and various changes may be made.
For example, as shown in Fig. 12, the postural change mat can be configured such that the head-use balloon 112 of the first embodiment and the trunk-use balloon formed by small balloons such as 113c, 113d, 113e, 113f and 113g are dispersedly arranged in the mat 200. In this embodiment, the balloons 113c ~ 113g are two-dimensionally and dispersedly arranged. In this embodiment, balloons 113c ~ 113g are dispersedly arranged two-dimensionally. When the balloons in the postural change mat 200 of the present embodiment are operated as shown in Fig. 13, the anteverted angle and the recurvate angle of the cervical spine can be made larger than the first embodiment.

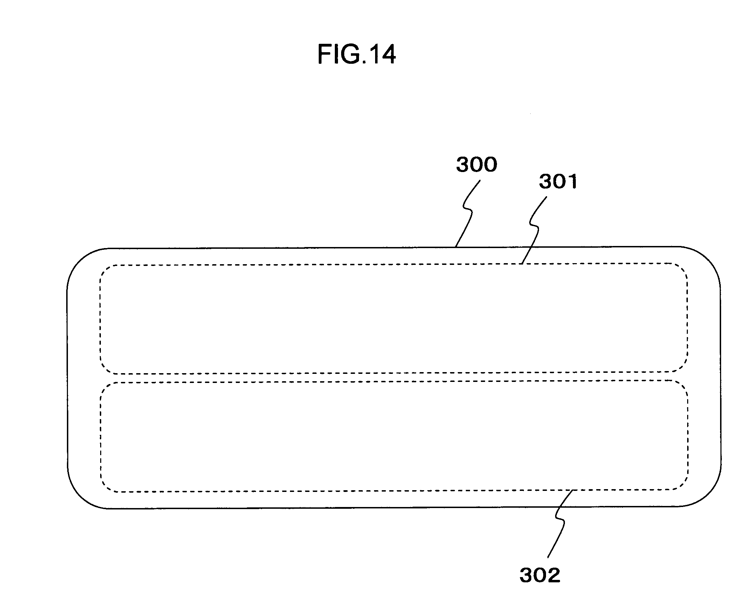

The form of the postural change mat may also be as shown in Fig. 14. In the postural change mat 300 shown in Fig. 14, balloons 301 and 302 are arranged in the mat along the longitudinal direction. By supplying air to the balloons 301 and 302 of the postural change mat individually, it is possible to tilt the object in the direction orthogonal to the body axis. Also, the object can be rolled in the direction orthogonal to the body axis by supplying/exhausting air to/from the balloons 301 and 302 alternately.

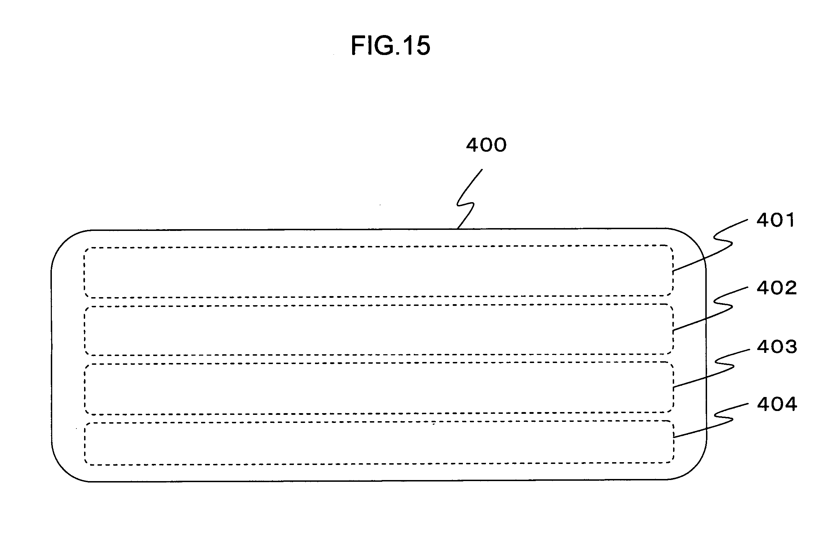

The postural change mat shown in Fig. 14 can be varied to the embodiment as shown in Fig. 15. The postural change mat 400 shown in Fig. 15 is configured such that the balloons 401, 402, 403 and 404 are dispersedly arranged in the mat in the longitudinal direction. By using the postural change mat 400, the object can be tilted or rolled over to the direction orthogonal to the body axis more stably compared to the postural change mat 300 shown in Fig. 14.

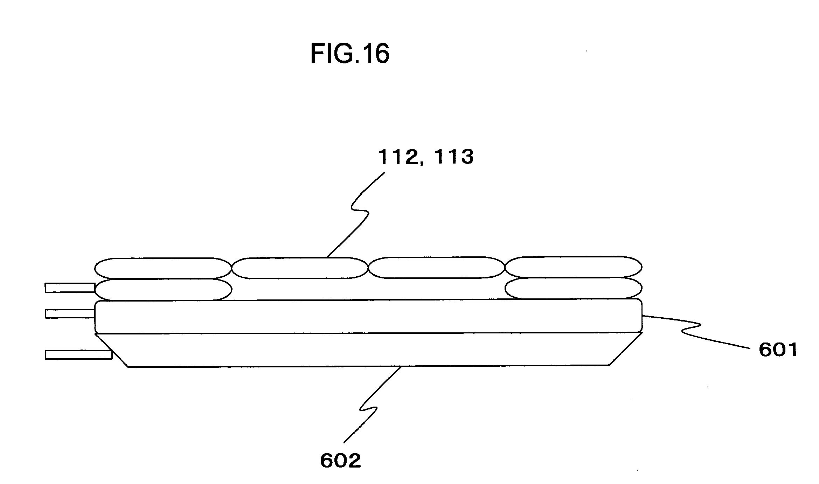

Also, in the case that a frame for supporting the top plate is provided to the table of the image diagnostic apparatus and the postural change mat of the present invention is to be applied to the top plate having the concaved form of a cross-section in the width direction, it may be configured so that the gap between the top plate supporting frame and the top plate can be filled by providing one or more steps of subsidiary air mats 601 and 602 in the lower side of the postural change mats 112 and 113 as shown in Fig. 16. In comparison with the air mat disclosed in Patent Document 3, the postural change mat of the present embodiment is capable of changing the posture of the imaging region of the object as desired by an examiner, since the object is supported directly by the postural change mat. According to the experiment, it is desirable in this example that the postural change mat is operated after sequentially expanding the subsidiary air mat to a predetermined height where the air mat reaches state of stability.

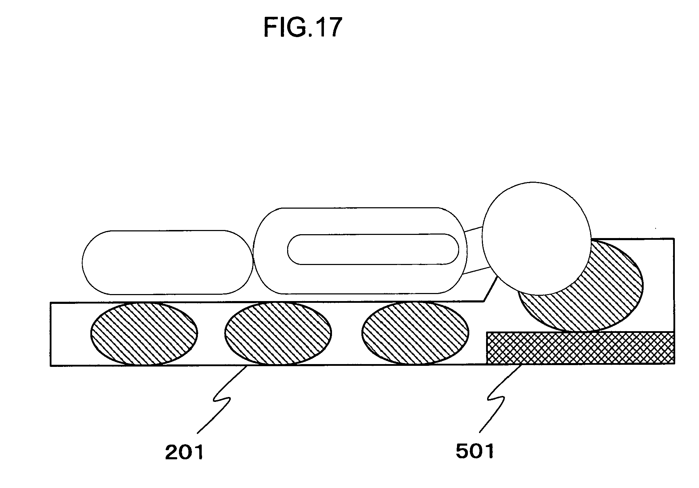

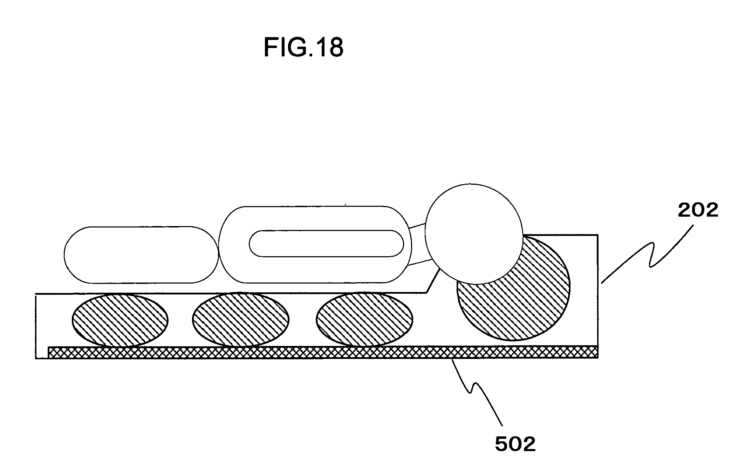

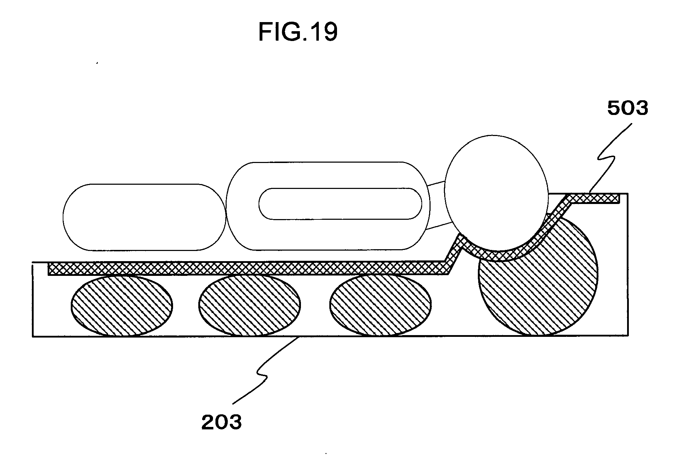

Also, the postural change device of the present invention can be configured to include an image data detector of an image diagnostic apparatus. For example, when it is assumed to combine the postural change device of the present invention and the MRI apparatus, it is possible to combine reception coils 501, 502 and 503 with the postural change mats 201, 202 and 203 as shown in Fig. 17 ~ Fig. 19. Furthermore, as an example for interlocking a planar detector in the lower side of the postural change mat as shown in Fig. 17 and Fig. 18, a two-dimensional planar sensor (flat panel detector) can be used as the detector in the case that an X-ray imaging device is used for an image diagnostic apparatus.

## Claims

1. A postural change device of an image diagnostic apparatus comprising:
a postural change mat having a bag-form mat and a plurality of balloons which expand/contract by supply/exhaust of air and are to be dispersedly arranged and firmly fixed inside the bag-form mat;
an air supply source for supplying/exhausting air to/from the balloons; and
a control unit for adjusting and controlling the supply/exhaust of air to/from the balloons.

2. The postural change device of the image diagnostic apparatus according to claim 1, wherein:
the mat has a form capable of placing an obj ect to be examined on a plane surface; and
the plurality of balloons are dispersedly arranged inside the mat so as to place an articular region of the object to be imaged in between them.

3. The postural change device of the image diagnostic apparatus according to claim 1, wherein the postural change mat comprises at least one subsidiary air mat in the lower part thereof.

4. The postural change device of the image diagnostic apparatus according to claim 2, wherein the plurality of balloons comprise:
a head-use balloon positioned at the head of the object; and
a trunk-use balloon positioned from the shoulder to the lower part of the object.

5. The postural change device of the image diagnostic apparatus according to claim 4, wherein the head-use balloon and the trunk-use balloon perform each operation of maximum expansion, intermediate degree of expansion and total deflation in the opposite direction for each other, by the air supply source and the control unit.

6. The postural change device of the image diagnostic apparatus according to claim 1, wherein:
the mat has a form capable of placing an object on a plane surface, and
the plurality of balloons are formed by at least two elongated balloons arranged along the body axis direction of the object.

7. The postural change device of the image diagnostic apparatus according to claim 6, wherein the at least two elongated balloons are alternately expanded and contracted by the air supply source and the control unit.

8. The postural change device of the image diagnostic apparatus according to claim 1, wherein the air supply source is a compressor.

9. The postural change device of the image diagnostic apparatus according to claim 1, wherein the control unit comprises:
a valve body disposed between the plurality of balloons and the air supply source, capable of supplying/exhausting air to the respective balloons individually or simultaneously; and
an operation panel for indicating the operation of the balloons which correspond to the operation of the valve body.

10. The postural change device of the image diagnostic apparatus according to claim 9, **characterized in that** the control unit is provided to the postural change mat.

11. The postural change device of the image diagnostic apparatus according to claim 9, **characterized in that** the valve body and the operation panel of the control unit are formed by non-magnetic material.

12. The postural change device of the image diagnostic apparatus according to claim 9, wherein the valve body is capable of switching air supply/exhaust operations between the operation for supplying/exhausting air to/from one balloon and the operation for exhausting air from the balloon and supplying air to the other balloons.

13. The postural change device of the image diagnostic apparatus according to claim 9, **characterized in that** the valve body comprises a hand valve that corresponds to the number of balloons that operate individually.

14. The postural change device of the image diagnostic apparatus according to claim 1, wherein the control unit comprises:
a control unit for performing drive control of the valve body; and
an interface for exchanging signals between the control unit and a controller of an image diagnostic apparatus.

15. The postural change device of the image diagnostic apparatus according to claim 14, wherein the control unit transmits a completion signal of the imaging preparation in one condition of the postural change device to a controller of an image diagnostic apparatus via an interface, and also receives an imaging completion signal in the previous condition of the postural change device from an image diagnostic apparatus via an interface.

16. The postural change device of the image diagnostic apparatus according to claim 15, wherein the completion signal of the imaging preparation for the postural change device is generated by measuring a predetermined elapsed time from the start of air supply/exhaust to/from a balloon by a timer provided to the control unit.

17. The postural change device of the image diagnostic apparatus according to claim 1, **characterized in that** a reception coil of an MRI apparatus is provided to the postural change mat.

18. The postural change device of the image diagnostic apparatus according to claim 1, **characterized in that** a two-dimensional planar sensor of an X-ray imaging apparatus is provided to the postural change mat.
